# EUROPEAN PATENT SPECIFICATION

(11) **EP 3 166 977 B1**
(45) Date of publication and mention of the grant of the patent: **11.11.2020**
(21) Application number: 14748135.2
(22) Date of filing: 07.07.2014
(51) Int. Cl.: C08B 31/00, C08L 3/02, A61K 47/00

(54) **A POLYMER BASED ON A MALTODEXTRIN FOR ENCAPSULATING ORGANIC COMPOUNDS**
POLYMER AUF DER BASIS VON MALTODEXTRIN ZUR VERKAPSELUNG ORGANISCHER VERBINDUNGEN
POLYMÈRE À BASE D'UNE MALTODEXTRINE POUR L'ENCAPSULATION DE COMPOSÉS ORGANIQUES

(43) Date of publication of application: 17.05.2017
(73) Proprietor: Roquette Italia S.p.A., 15063 Cassano Spinola (IT)
(72) Inventor: TROTTA, Francesco, I-14100 Asti (IT); FOSSATI, Ernesto, I-15063 Gavazzana (IT)
(74) Representative: Cattaneo, Elisabetta
(86) International application number: PCT/EP2014/064466
(87) International publication number: WO 2016/004974

(56) References cited:
- EP-A1- 2 540 773
- WO-A1-2009/003656
- WO-A1-2009/153798
- WO-A1-2012/147069

## Description

### FIELD OF THE INVENTION

The present invention relates to a polymer based on a maltodextrin for encapsulating organic compounds in different industrial fields such as in food and drink fields, in pharmaceutical applications and in insecticidal applications.

### BACKGROUND OF THE INVENTION

In many fields of the industry such as food and drink fields, wherein labile organic molecules are used, it is necessary to encapsulate, complex or trap these molecules in order to avoid their degradation or their interaction with agents or environmental conditions capable to convert the molecules to uninteresting compounds or even to destruct them.

In the pharmaceutical or insecticidal fields the encapsulation techniques are used in order to release the pharmaceutical/insecticidal substances in a specific way so as to release them where and when needed.

Therefore, different encapsulating techniques, through which these organic compounds are packaged in an appropriate manner in order to guarantee both good preservation without alteration of their properties and their availability, are known.

Among the known encapsulating agents cyclodextrins and their derivatives, also as a mixture, are used.

For instance, EP1304044 describes the use of sugars, modified starches, maltodextrins and other polymers in combination with hydroxypropylcellulose to provide a matrix capable to encapsulate a flavour or a fragrance material. Cyclodextrins are also used in very different technical fields to encapsulate organic molecules, by forming inclusion complexes or supramolecular complex with interesting organic substances.

For example, in the literature and in patent applications insecticide, acaricide, fungicide, snailcide and vermicide formulations in cyclodextrins (CD) are described (see for example Szente, L. et al, "Cyclodextrins in Pesticides", in "Comprehensive Supramolecular Chemistry" , pages 503-514 , Elsevier (1996); Castillo, J.A. et al., Drug Develop. Ind. Pharm. 1999, 25, 1241-1248; Lezcano, M. et al., J. Agric. Food Chem. 2002, 50, 108-112). The main purposes of said supramolecular complexes are: modification of the physico-chemical properties of active principles without however altering their biological activity once the active principles are released, greater stability, increased wettability and bioavailability of poorly soluble and difficultly absorbable active principles, reduced environmental toxicity and reduced toxicity for operators.

The α,β,γ cyclodextrins are natural or semi-synthetic cyclic oligosaccharides, being generally biodegradable; β-CD, γ-CD and certain derivatives thereof such as hydroxypropyl-β-cyclodextrin (HP-β-CD) and sulfobutyl ether-β-cyclodextrin (SBE-β-CD) are mostly used in industrial applications. However, the use of cyclodextrins is highly regulated.

In WO2013/179196 a process for the treatment of beverages is described, wherein polymers of α,β,γ cyclodextrins are used. The polymers resulted to stabilize the protein fractions in wine.

Alternatively, the use of starches rich in amylose (starches containing more than 50% amylose) was proposed for encapsulation, but it involves various constraints, since they require very strict conditions of preparation and use. Indeed, these starches retrograde rapidly due to their richness in amylose.

It is also known to stabilize the starch. The stabilization is obtained by substitution of the hydroxyl functions of the starch, by esterification or etherification. It can also be obtained by oxidation. These stabilization treatments are in particular hydroxypropylation, acetylation, phosphation and oxidation. These reactions addressed to the stabilization, even if they allow to reduce the retrogradation temperature of the starch, do reduce their ability to form inclusion complexes.

In the field of wine, the document EP 820 702 describes the use of a pea starch as an encapsulation agent by spray-drying or by lyophilization. The presence of long polysaccharidic chains is the characteristic essential for the pea starch to be able to encapsulate the flavourings according to the described invention.

In US2010/0196542 the use of a maltodextrin and/or a glucose syrup for the encapsulation of organic compounds is described, wherein the maltodextrin and/or the glucose syrup are obtained from a leguminous starch having an amylose content comprised between 25% and 50%, expressed as dry weight relative to the dry weight of starch. As explained in such a document, amylose is organized in helices with a hydrophilic external surface due to the presence of hydroxyl groups and with a hydrophobic internal surface due to the presence of hydrogen atoms. This helical structure confers the amylose the necessary characteristics for the encapsulation of active principles or flavourings, even if the use of pure amylose cannot however be envisaged on an industrial scale due to their great propensity to crystallization or retrogradation.

The maltodextrin used in US2010/0196542 resulted to be an alternative product not only for starch rich in amylose but also for the cyclodextrins. With respect to the starch rich in amylose, the maltodextrins of US2010/0196542 resulted to be soluble in cold water and easy to use for the encapsulation, particularly in the industry of wine. On the other hand, the maltodextrins of US2010/0196542 resulted to demonstrate high yields of encapsulation, particularly of flavourings, when compared with cyclodextrins without being subjected to strict use regulations.

It is felt the need to provide further encapsulating agents having good and improved inclusion/encapsulation capability including a controlled release of the included/encapsulated organic compound, and easiness of use in all solvents, while being safe.

### SUMMARY OF THE INVENTION

During experimentation addressed to study maltodextrins the Applicant has surprisingly found out that a specific modification of the maltodextrin deriving from starch comprising amylose in the range from 25 to 50% allows to obtain a product capable to be extremely stable to relatively high temperatures, very good solubilizing properties and high stability of the formed complexes with the organic substances. Therefore the present invention relates to a cross-linked polymer obtainable by reacting a maltodextrin deriving from starch comprising amylose in the range from 25 to 50% expressed as dry weight relative to the dry weight of the starch and at least one cross-linking compound having a electropositive carbon atom selected from the group consisting of a dicarboxylic acid, dianhydrides, carbonyldiimidazole, diphenylcarbonate, triphosgene, acyl dichlorides, diisocyanates, diepoxides, wherein the maltodextrin derives from leguminous starch and the leguminous plant is chosen from the group consisting of pea, bean, broad bean, horse bean and their mixtures.

In a preferred embodiment the present invention concerns to a cross-linked polymer obtainable by reacting a maltodextrin deriving from starch comprising amylose in the range from 25 to 50% expressed as dry weight relative to the dry weight of the starch and at least one compound selected from the group consisting of a dicarboxylic acid, a dianhydride, carbonyldiimidazole and a diisocyanate.

In the more preferred embodiment, the cross-linked polymer according to the invention is obtainable by reacting a maltodextrin deriving from starch comprising amylose in the range from 25 to 50% expressed as dry weight relative to the dry weight of the starch and at least one cross-linking agent selected from pyromellitic dianhydride, 1,1'-carbonyldiimidazole, hexamethylene diisocyanate, citric acid and tartaric acid.

In a still more preferred embodiment, the cross-linked polymer according to the invention is obtainable by reacting a maltodextrin deriving from starch comprising amylose in the range from 25 to 50% expressed as dry weight relative to the dry weight of the starch and a cross-linking compound selected from 1,1'-carbonyldiimidazole and hexamethylene diisocyanate.

The cross-linked polymers of the invention are in the form of nano-porous material capable to strongly encapsulate/trap/include organic substances. Furthermore the polymers of the invention are advantageously insoluble in water and in all the organic solvent, so thus to be used as encapsulating agent in a solid form.

### DESCRIPTION OF THE FIGURES:

Figure 1 shows the spectrum of TGA analysis of the cross-linked polymer of example 1.
Figure 2 shows the spectrum of ATR-FTIR analysis of the cross-linked polymer of example 1.
Figure 3 shows the spectrum of TGA analysis of the cross-linked polymer of example 2.
Figure 4 shows the spectrum of ATR-FTIR analysis of the cross-linked polymer of example 2.
Figure 5 shows the spectrum of TGA analysis of the cross-linked polymer of example 3.
Figure 6 shows the spectrum of ATR-FTIR analysis of the cross-linked polymer of example 3.
Figure 7 shows the spectrum of ATR-FTIR analysis of the cross-linked polymer of example 4.
Figure 8 shows the spectrum of ATR-FTIR analysis of the cross-linked polymer of example 5.
Figure 9 shows the spectrum of ATR-FTIR analysis of the cross-linked polymer of example 6.
Figure 10 shows the spectrum of ATR-FTIR analysis of the cross-linked polymer of example 7.
Figure 11 shows the results of the absorption of methyl orange of the polymer of example 2 and example 3 through the UV-Vis analysis.
Figure 12 shows the results of the absorption of the polymer of example 2 to a 5ml of a solution of anisole (2.45x10⁻⁴ M) through the UV-Vis analysis.

### DETAILED DESCRIPTION OF THE INVENTION

The present invention hence relates to a cross-linked polymer obtainable by reacting a maltodextrin deriving from starch comprising amylose in the range from 25 to 50% expressed as dry weight relative to the dry weight of the starch and at least one cross-linking compound having a electropositive carbon atom selected from the group consisting of a dicarboxylic acid, dianhydrides, carbonyldiimidazole, diphenylcarbonate, triphosgene, acyl dichlorides, diisocyanates and diepoxides., wherein the maltodextrin derives from leguminous starch and the leguminous plant is chosen from the group consisting of pea, bean, broad bean, horse bean and their mixtures.

The cross-linked polymer of the invention is hence obtainable from a maltodextrin deriving from starch comprising amylose in the range from 25 to 50% expressed as dry weight relative to the dry weight of the starch.

The maltodextrin of the invention derives from leguminous starch. By "leguminous" is meant within the meaning of the present invention any plant belonging to the families of the Caesalpiniaceae, Mimosaceae or Papilionaceae and notably any plant belonging to the family of the Papilionaceae such as, for example, pea, bean, broad bean, horse bean, lentil, lucerne, clover or lupin. This definition includes in particular all the plants described in any one of the tables contained in the article by R. HOOVER et al., 1991 (HOOVER R. (1991) "Composition, structure, functionality and chemical modification of leguminous starches: a review" Can. J. Physiol. Pharmacol., 69, pp.: 79-92). The leguminous plant is chosen from the group formed by the pea, bean, broad bean, horse bean and their mixtures. According to a preferred and advantageous embodiment, the leguminous plant is a variety of pea or horse bean, producing seeds containing at least 25%, preferably at least 40%, by weight of starch (dry/dry). More advantageously, said leguminous plant is the pea. The term "pea" being here considered in its broadest sense and including in particular: all the wild "smooth pea" varieties and all the mutant "smooth pea" and "wrinkled pea" varieties, irrespective of the uses for which said varieties are generally intended (human consumption, animal nutrition and/or other uses).

The leguminous starch of the invention preferably has an amylose content comprised between 30% and 40%, in particular comprised between 35% and 40%, and more preferably between 35% and 38%, these percentages being expressed as dry weight relative to the dry weight of starch.

The maltodextrins are conventionally obtained by acid and/or enzymatic hydrolysis of starch. Referring to the regulatory status, the maltodextrins have a dextrose equivalent (DE) of 1 to 20.

Preferably in the present invention the maltodextrin has a dextrose equivalent (DE) of 17 and an average molecular weight by weight of about 12000 D.

The cross-linked polymer is hence obtainable from reacting a crosslinking compound having a electropositive carbon atom selected from the group consisting of a dicarboxylic acid, dianhydrides, carbonyldiimidazole, diphenylcarbonate, triphosgene, acyl dichlorides, diisocyanates and diepoxides.

In the present invention when the definition "a compound having a electropositive carbon atom" is used is meant a compound having a carbon atom subjected to nucleophilic attack, i.e. having a partial positive charge.

Preferably, the at least one cross-linking compound having a electropositive carbon atom is selected from a dicarboxylic acid, a dianhydride, carbonyldiimidazole and a diisocyanate.

Among the dicarboxylic acids, in the present invention the following diacids can be used: polyacrylic acid, butane tetracarboxylic acid, succinic acid, tartaric acid and citric acid. More preferably the cross-linking compound having a electropositive carbon atom is citric acid. In an advantageous embodiment the cross-linked polymer is obtainable by using citric acid and tartaric acid as cross-linking agents.

Among the dianhydrides, in the present invention the following dianhydrides can be used: diethylenetriaminepentaacetic dianhydride, ethylenediaminetetraacetic dianhydride, benzophenone-3,3',4,4'-tetracarboxylic dianhydride, and pyromellitic dianhydride. More preferably the cross-linking compound having a electropositive carbon atom is pyromellitic dianhydride.

Among the acyl-chlorides, in the present invention the following acyl chlorides can be used: terephthaloyl chloride, sebacoyl chloride, succinyl chloride. More preferably the cross-linking compound having a electropositive carbon atom is terephthaloyl chloride.

Among the diisocyanates, in the present invention the following diisocyanates can be used: toluenediisocyanate, Isophorone diisocyanate, 1,4-Phenylene diisocyanate, Poly(hexamethylene diisocyanate), and hexamethylene diisocyanate. More preferably the cross-linking compound having a electropositive carbon atom is hexamethylene diisocyanate.

More preferably, the compound having a electropositive carbon atom is selected from pyromellitic dianhydride, 1,1'-carbonyldiimidazole, hexamethylene diisocyanate, citric acid and tartaric acid.

As it will be more evident from the following experimental part the polymer of the invention resulted to be stable to relatively high temperatures, to have high complexation capability, high solubilizing properties and high stability of the formed complexes. Furthermore the polymer of the invention have the advantage of being easy to obtain, presently with no particular problems of law regulations.

The present invention relates also to a process for preparing the cross-linked polymer of the invention, comprising the following step:
a) Preparing the solution of a maltodextrin deriving from starch comprising amylose in the range from 25 to 50% expressed as dry weight relative to the dry weight of the starch;
b) Adding at least one cross-linking compound having an electropositive carbon atom selected from the group of a dicarboxylic acid, dianhydride, carbonyldiimidazole, diphenylcarbonate, triphosgene, acyl dichloride, diisocyanate, cyclodextrin, diepoxide and a polyepoxide.
c) Obtaining the cross-linked polymer, wherein the maltodextrin derives from leguminous starch and the leguminous plant is chosen from the group consisting of pea, bean, broad bean, horse bean and their mixtures.

The molar ratio between the maltodextrin of step a) and the cross-linking compound having an electropositive carbon is preferably from 1:0.5 to 1:250, more preferably the molar ratio between the maltodextrin of step a) and the at least one cross-linking compound having an electropositive carbon is 1: 0.57, with respect to the glucose unit of the maltodextrin, i.e. 0.57 moles of cross-linker for each mole of glucose unit. In another embodiment the molar ratio between the maltodextrin of step a) and the at least one compound having an electropositive carbon is 1 : 3 or 1 :3.28 with respect to the glucose unit of the maltodextrin, i.e. 3 or 3.28 moles of cross-linker for each mole of glucose unit.

Advantageously, the process of the invention provides for adding a cyclodextrin to the solution of step a) together with the cross-linking agent of step b). Among the cyclodextrins, α-cyclodextrin,β-cyclodextrin and γ cyclodextrin can be used. More preferably β-cyclodextrin is added.

In the embodiment of the invention wherein the polymer is obtainable by a crosslinking compound of both citric acid and tartaric acid, the molar ratio between the maltodextrin of step a) and tartaric acid and citric acid is 1:1:2, i.e. one mole of tartaric acid and two moles of citric acid for each mole of glucose of the maltodextrin by considering the glucose unit with a molar mass (molecular weight) of 180.15 g/mol.

The solution of step a) is preferably carried out with dimethyl sulfoxide or with N,N-dimethylformamide, N-methylpyrrolidinone.

The polymer of the invention can be used as encapsulating agent. The polymer can be used in the pharmaceutical industry, the cosmetic industry, the food industry, the paper and non-wovens industry, textiles, super-odoriferous products and deodorants, detergents or phytosanitary products, in drink industry and insecticidal field.

The polymer of the invention allows encapsulation/inclusion/entrapment of various organic compounds with different physicochemical characteristics and sizes, such as drugs, dyes, gases, vapors.

In a further aspect the invention hence concerns the use of the cross-linked polymer of the invention for encapsulation/inclusion/entrapment of an organic compound.

In a still further aspect the invention relates a method of encapsulation/inclusion of an organic compound. For example, the polymer of the invention can be used not in a water dissolved state, but in the solid state. In this case, the polymer is mixed with a small amount of water, insufficient to dissolve it completely but sufficient to allow to obtain a paste. This paste is then mixed, by kneading and/or mixing, with the compound to be encapsulated, in a powder state or in a dissolved state in an appropriate solvent. Alternatively, the inclusion compound can be easily obtained by adding the selected amount of cross linked polymer with an excess of guest molecule dissolved in suitable solvent after stirring overnight at room temperature the encapsulation occurs and it is recovered by simply filtration under vacuum. The invention will be now described with reference to examples of preparation of the polymer of the invention and examples of encapsulation/inclusion of organic compounds.

### Experimental part

### Example 1: Preparation of the cross-linked polymer of the invention by reacting a maltodextrin deriving from starch comprising amylose in the range from 25 to 50% expressed as dry weight relative to the dry weight of the starch and pyromellitic dianhydride as cross-linking agent.

4.89 g of maltodextrin sold as Kleptose Linecaps 17 from Roquette Italia SpA, having DE of 17 (dried in stove at 100-120°C for at least one night) were solubilized under continued stirring in 20 ml of dimethyl sulfoxide in a 100ml flask. 5ml of triethylamine were then added and, after a few minutes, 3.76 g of pyromellitic dianhydride were added. The molar ratio between the initial maltodextrin and pyromellitic dianhydride was 1:0.57 expressed as molar ratio of one mole of glucose of the maltodextrin with respect to 0.57 moles of pyromellitic dianhydride. After a short time, the reticulation process blocked the stir bar. After 24 hours the reaction was considered complete. In the following days, the polymer was ground in a mortar and washed with deionized water in a Buchner funnel with water jet pump. After the air drying, the polymer was purified in a Soxhlet extractor with acetone for a total time of about 14 hours.

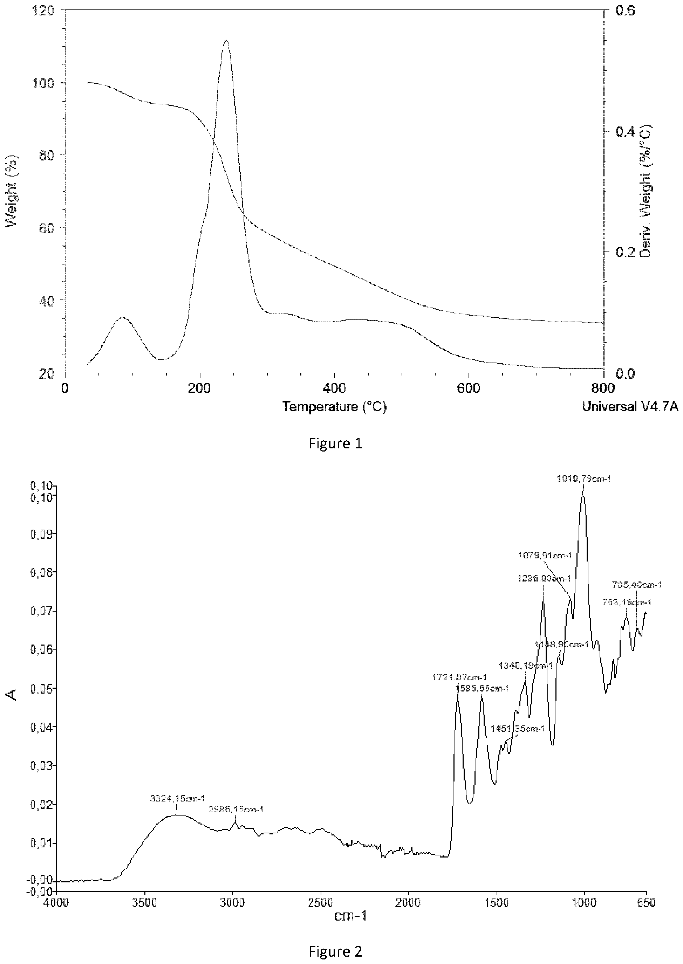

The cross-linked polymer so obtained was analysed by TGA analysis, using a TA Instruments TGA2050 v5.4A, with ramp of 10°C per minute in N₂. The result of the analysis is the thermogram reported in Figure 1. The first weight loss (∼6%) that occurred between 50 and 100°C is mainly due to the moisture absorbed on the sample. The degradation of the polymeric structure started around 150°C and continued till 600°C, nevertheless, the maximum degradation rate was reached at 240°C. At 800°C, a final residue of about 20% was observed.

Furthermore in order to better characterize the cross-linked polymer of the invention, it was analyzed with ATR-FTIR analysis, employing a PerkinElmer Spectrum 100 FT-IR spectrometer. The result of the analysis is the spectrum reported in Figure 2. In the infrared spectrum, the peaks of the carbonyl groups (i.e. 1721, 1585 cm⁻¹), introduced by the cross-linker, can be observed in addition to the characteristic bands of the maltodextrin units. In the following table the main peaks are listed, along with the corresponding absorbing groups.

| **Wave number (cm⁻¹)** | **Absorbing group** |
|---|---|
| 3600-3100 | O-H |
| 2990-2800 | C-H |
| 1721 | C=O in carboxylic moieties |
| 1585 | C=O in carboxylate groups |
| 1236 | C-O |
| 1010 | C-O |

The polymer obtained was also subjected to CHNS analysis, in a Thermoscientific FlashEA 1112 Series instrument. The results are reported in the table below:

| | % N | % C | % H | % S |
|---|---|---|---|---|
| Cross-linked polymer of example 1 | 2.76 | 50.20 | 6.33 | 0.00 |

### Example 2: Preparation of the cross-linked polymer of the invention by reacting a maltodextrin deriving from starch comprising amylose in the range from 25 to 50% expressed as dry weight relative to the dry weight of the starch and 1,1'-carbonyldiimidazole as cross-linking agent

4.89 g of maltodextrin sold as Kleptose Linecaps 17 from Roquette Italia SpA, having DE of 17 (dried in stove at 100-120°C for at least one night) were solubilized under continued stirring in 20 ml of anhydrous N,N-dimethylformamide in a 100ml flask. 2.79 g of 1,1'-carbonyldiimidazole were then added. The molar ratio between the initial maltodextrin and 1,1'-carbonyldiimidazole was 1:0.57 expressed as molar ratio of one mole of glucose of the maltodextrin with respect to 0.57 moles of 1,1'-carbonyldiimidazole. The obtained mixture was heated in an oil bath till a temperature of 90°C was reached. After a few minutes. the reticulation process blocked the stir bar. The heating has continued for at least 2-3 hours so as to complete the crosslinking reaction. In the following days, the polymer was ground in a mortar and washed with deionized water in a Buchner funnel with water jet pump. After the air drying, the polymer was purified in a Soxhlet extractor with ethanol for a total time of about 14 hours.

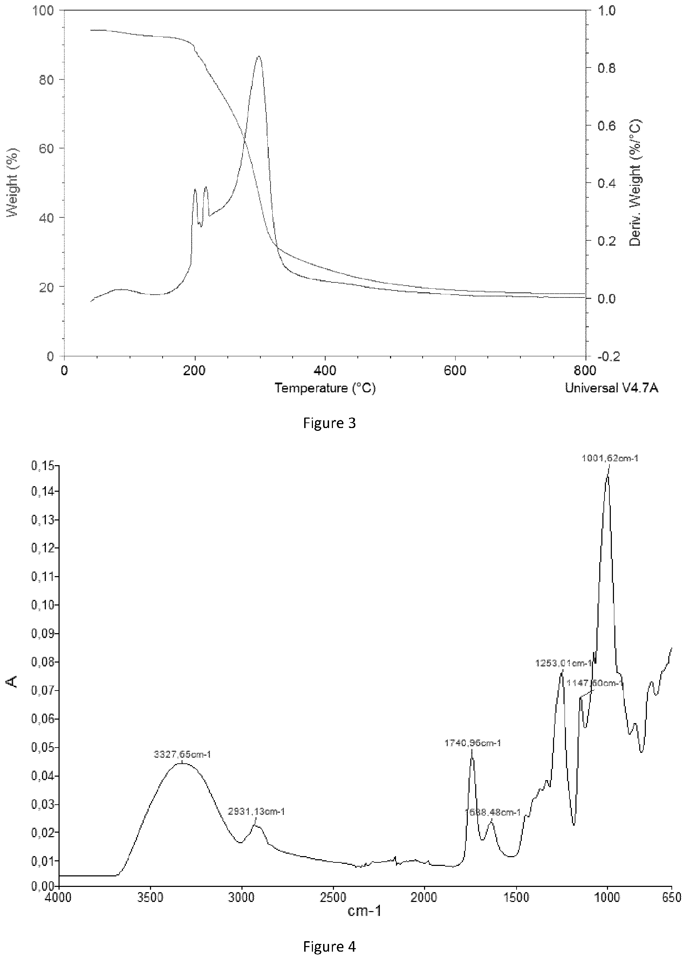

The cross-linked polymer so obtained was analysed by TGA analysis, using a TA Instruments TGA2050 v5.4A, with ramp of 10°C per minute in N₂. The result of the analysis is the thermogram reported in Figure 3. The initial weight loss (∼2%) comprised between 50 and 100°C can be attributed to the release of absorbed moisture. The thermal degradation of the polymer started approximately at 175°C and led to a final residue of about 18% at 800°C. The maximum degradation rate was observed at 298 °C.

Furthermore in order to better characterize the cross-linked polymer of the invention, it was analyzed with ATR-FTIR analysis, employing a PerkinElmer Spectrum 100 FT-IR spectrometer. The result of the analysis is the spectrum reported in Figure 4. The principal absorption signals are listed in the table presented below. Additionally to the main absorption bands of the glucose units (i.e. stretching and bending of O-H groups at 3600-3100 cm⁻¹ and 1638 cm⁻¹, respectively, C-H stretching around 2900 c m⁻¹ and the stretching of C-O bonds in alcohol, ester and ether groups in the range 1260-1000 cm⁻¹) the characteristic absorption peak of carbonyl moieties appears at 1741 cm⁻¹, thus confirming the presence of the cross-linking bridges in the polymer structure.

| **Wave number (cm⁻¹)** | **Absorbing group** |
|---|---|
| 3600-3100 | O-H |
| 2990-2800 | C-H |
| 1741 | C=O |
| 1638 | O-H |
| 1253 | C-O |
| 1002 | C-O |

The polymer obtained was also subjected to CHNS analysis in a Thermoscientific FlashEA 1112 Series instrument. The results are reported in the table below:

| | % N | % C | % H | % S |
|---|---|---|---|---|
| Cross-linked polymer of example 2 | 0.34 | 39.33 | 5.61 | 0.00 |

### Example 3: Preparation of the cross-linked polymer of the invention by reacting a maltodextrin deriving from starch comprising amylose in the range from 25 to 50% expressed as dry weight relative to the dry weight of the starch and hexamethylenediisocyanate carbonyldiimidazole as cross-linking agent

4.89 g of maltodextrin sold as Kleptose Linecaps 17 from Roquette Italia SpA, having DE of 17 (dried in stove at 100-120°C for at least one night) were solubilized under continued stirring in 20 ml of dimethyl sulfoxide in a 100ml flask. 0.5 g of 1,4-diazabicyclo[2.2.2]octane were then added and, after a few minutes, 2.77 g of hexamethylenediisocyanate were added. The molar ratio between the initial maltodextrin and hexamethylendiisocyanate was 1 :0.57 expressed as molar ratio of one mole of glucose of the maltodextrin with respect to 0.57 moles of hexamethylendiisocyanate. After a short time, the reticulation process blocked the stir bar. After 24 hours the reaction was considered complete. In the following days, the polymer was ground in a mortar and washed with deionized water in a Buchner funnel with water jet pump. After the air drying, the polymer was purified in a Soxhlet extractor with acetone for a total time of about 14 hours.

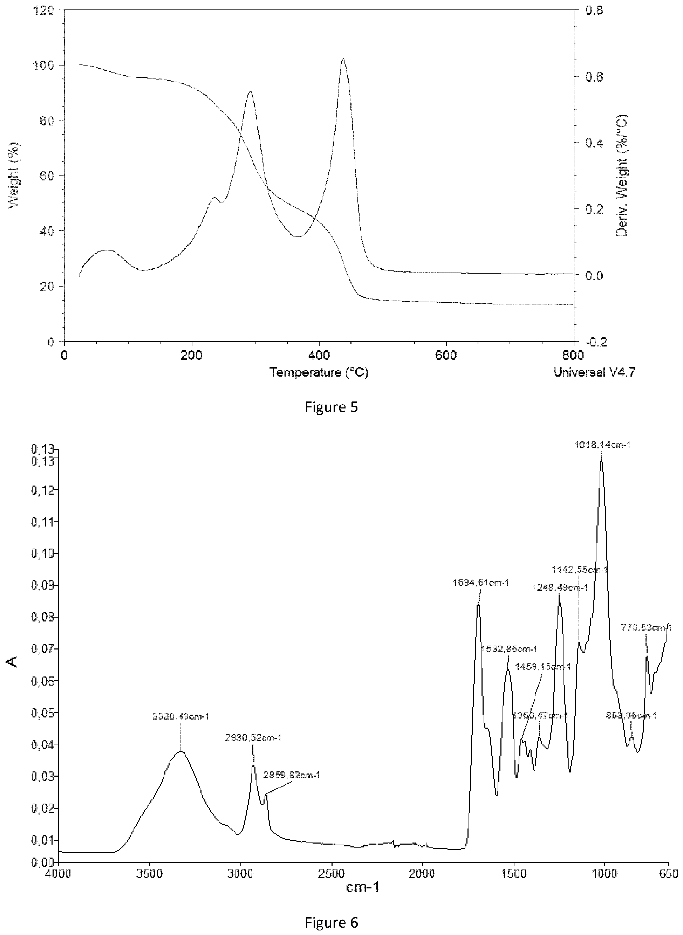

The cross-linked polymer so obtained was analysed by TGA analysis, using a TA Instruments TGA2050 v5.4A, with ramp of 10°C per minute in N₂. The result of the analysis is the thermogram reported in Figure 5. The amount of absorbed moisture (∼5%) was lost in the first step of the heating program, comprised between 40 and 120°C. The polymer was proved to be stable up to 150°C, then degradation occurred through a multi-step process, in which three main weight losses can be observed. The maximum degradation rates of the three processes are placed at 236, 291 and 438°C, respectively. Finally, a residue of 13% was registered at 800°C.

Furthermore in order to better characterize the cross-linked polymer of the invention, it was analyzed with ATR-FTIR analysis, employing a PerkinElmer Spectrum 100 FT-IR spectrometer. The result of the analysis is the spectrum reported in Figure 6. The principal absorption peaks are summarized in the subsequent table.

| **Wave number (cm⁻¹)** | **Absorbing group** |
|---|---|
| 3600-3100 | O-H |
| 2990-2800 | C-H, N-H |
| 1695 | C=O |
| 1533 | N-H |
| 1248 | C-O |
| 1018 | C-O |

The most intense signals associated with the maltodextrin units are located in the ranges 3600-3100 cm⁻¹ and 1260-1000 cm⁻¹ and they are mostly due to the stretching vibrations of O-H and C-O bonds, respectively. The presence of urethane units is proved by the absorption peaks at 1695 and 1533 cm⁻¹, caused by the stretching of carbonyl groups and the bending vibrations of N-H bonds, respectively. Whereas the absorption band comprised between 2990 and 2800 cm⁻¹ can be attributed to the stretching vibrations of C-H bonds, carried by both maltodextrin and cross-linker units, overlaid with the stretching vibrations of the urethane N-H bonds.

The polymer obtained was also subjected to CHNS analysis in a Thermoscientific FlashEA 1112 Series instrument. The results are reported in the table below:

| | % N | % C | % H | % S |
|---|---|---|---|---|
| Cross-linked polymer of example 3 | 0.34 | 39.33 | 5.61 | 0.00 |

### Example 4: Preparation of the cross-linked polymer of the invention by reacting a maltodextrin deriving from starch comprising amylose in the range from 25 to 50% expressed as dry weight relative to the dry weight of the starch and Citric acid as cross-linking agent

4.00 g of maltodextrin sold as Kleptose Linecaps 17 from Roquette Italia SpA, having DE of 17, 1.00 g of sodium hypophosphite monohydrate (NaH₂PO₂•H₂O) and 14.22 g of citric acid were added under stirring in 20 ml of deionized water. The molar ratio between the maltodextrin and citric acid was 1:3 expressed as molar ratio of one mole of glucose of the maltodextrin with respect to 3 moles of citric acid. The solution was then heated at 100°C until it was clear (about 5 minutes). The solution was then poured in a Petri dish, then maintained in stove at a temperature of about 80°C and low pressure (about 80 mbar) till the compound resulted to be dried. (requested time of about 10 days). At the end, the polymer so obtained was ground through a pestle mortar and washed with deionized water in excess either through filtration on a Buchner apparatus or through repeated centrifugation cycles until the washing water resulted to be colourless. The last washing cycles with the funnel and/or centrifuge were carried out by adding acetone instead of water to the polymer in order to accelerate the drying process of the polymer. The treatment with acetone was prolonged until the washing solvent was colourless. The polymer was then left to dry at the open air for a few days.

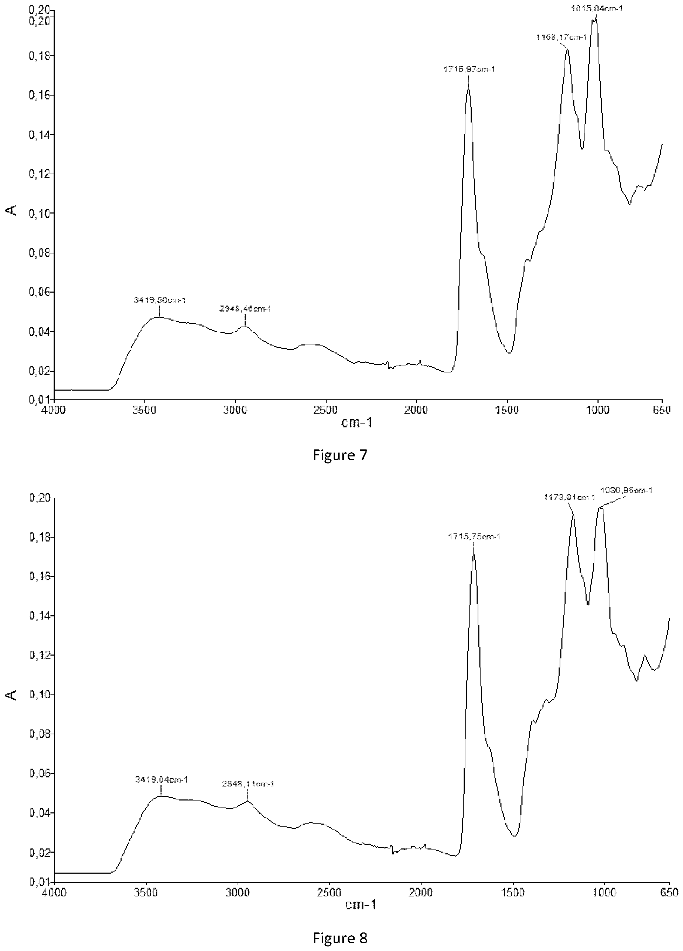

In order to characterize the cross-linked polymer of the invention, it was analyzed with ATR-FTIR analysis, employing a PerkinElmer Spectrum 100 FT-IR spectrometer. The result of the analysis is the spectrum reported in Figure 7.

The characteristic peak of the carbonyl groups, carried by the citric acid cross-linking units, was observed at 1716 cm⁻¹. Aside from it, the major absorption signals, deriving from both maltodextrins and citric acid units are listed in the subsequent table. The infrared spectrum confirmed the expected composition of the polymer.

| **Wave number (cm⁻¹)** | **Absorbing group** |
|---|---|
| 3600-3100 | O-H |
| 2990-2800 | C-H |
| 1716 | C=O |
| 1168 | C-O |
| 1015 | C-O |

The polymer obtained was also subjected to CHNS analysis in a Thermoscientific FlashEA 1112 Series instrument. The results are reported in the table below:

| | % N | % C | % H | % S |
|---|---|---|---|---|
| Cross-linked polymer of example 4 | 0.00 | 43.13 | 4.42 | 0.00 |

### Example 5: Preparation of the cross-linked polymer of the invention by reacting a maltodextrin deriving from starch comprising amylose in the range from 25 to 50% expressed as dry weight relative to the dry weight of the starch and Citric acid as cross-linking agent

The same procedure and ingredients stated in Example 4 was repeated by using an amount of 15.54 g of citric acid. In this case the molar ratio between the maltodextrin and citric acid was 1:3.28 expressed as molar ratio of one mole of glucose of the maltodextrin with respect to 3.28 moles of citric acid
In order to characterize the cross-linked polymer of the invention, it was analyzed with ATR-FTIR analysis, employing a PerkinElmer Spectrum 100 FT-IR spectrometer. The result of the analysis is the spectrum reported in Figure 8.

The absorption peak that appeared at 1716 cm⁻¹, caused by the stretching vibrations of carbonyl groups, clearly indicated the presence of the citric acid cross-linking units. Along with the carbonyl signal, the principal peaks, which were due to the vibrations of both maltodextrin and cross-linking units, are listed in the table below.

| **Wave number (cm⁻¹)** | **Absorbing group** |
|---|---|
| 3600-3100 | O-H |
| 2990-2800 | C-H |
| 1716 | C=O |
| 1173 | C-O |
| 1031 | C-O |

The polymer obtained was also subjected to CHNS analysis in a Thermoscientific FlashEA 1112 Series instrument. The results are reported in the table below:

| | % N | % C | % H | % S |
|---|---|---|---|---|
| Cross-linked polymer of example 5 | 0.00 | 39.14 | 4.21 | 0.00 |

### Example 6: Preparation of the cross-linked polymer of the invention by reacting a maltodextrin deriving from starch comprising amylose in the range from 25 to 50% expressed as dry weight relative to the dry weight of the starch and citric acid and tartaric acid as cross-linking agent

4.00 g of maltodextrin sold as Kleptose Linecaps 17 from Roquette Italia SpA, having DE of 17, 1.00 g of sodium hypophosphite monohydrate (NaH₂PO₂•H₂O), 3.7 g of tartaric acid and 9.48 g of citric acid were added under stirring in 20 ml of deionized water. The molar ratio between the maltodextrin, tartaric acid and citric acid was 1:1:2 expressed as molar ratio of one mole of glucose of the maltodextrin with respect to 1 mole of tartaric acid and 2 moles of citric acid. The solution was then heated at 100°C until it was clear (about 5 minutes). The solution was then poured in a Petri dish, then maintained in stove at a temperature of about 80°C and a pressure of about 80 mbar for ten days till the compound resulted to be dried. At the end, the polymer so obtained was ground through a pestle mortar and washed with deionized water in excess either through a Buchner funnel or through repeated centrifugation cycles. The treatment was continued until the washing water resulted to be colourless. The last washing cycles with the funnel and/or centrifuge were carried out by adding acetone instead of water. The treatment with acetone was prolonged until the washing solvent was colourless. The polymer was then left to dry at the open air for a few days.

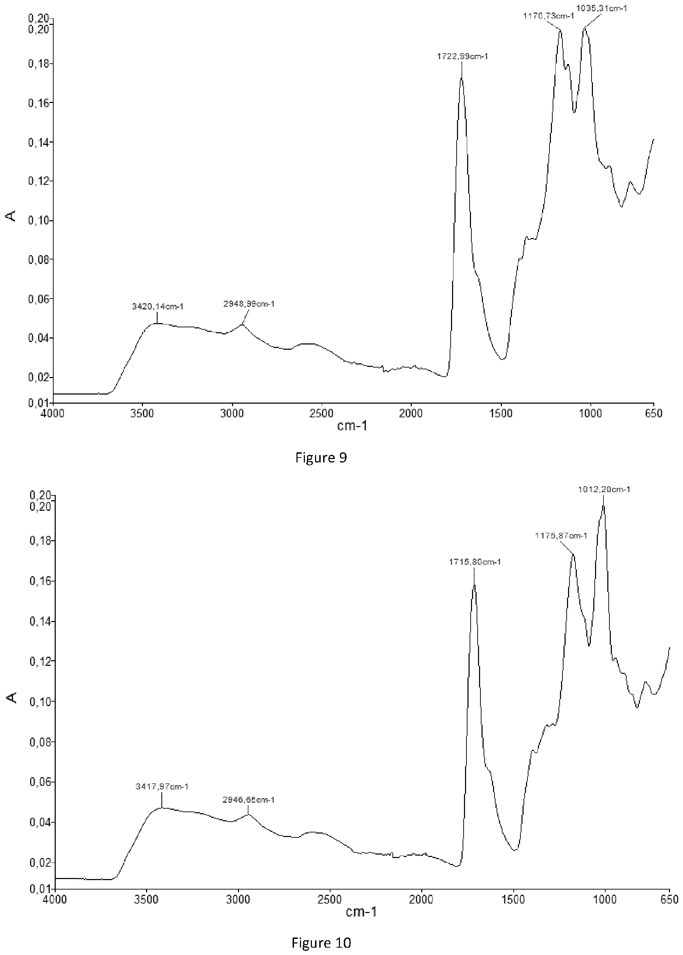

In order to characterize the cross-linked polymer of the invention, it was analyzed with ATR-FTIR analysis, employing a PerkinElmer Spectrum 100 FT-IR spectrometer. The result of the analysis is the spectrum reported in Figure 9. The principal absorption peaks are summarized in the subsequent table

| **Wave number (cm⁻¹)** | **Absorbing group** |
|---|---|
| 3600-3100 | O-H |
| 2990-2800 | C-H |
| 1723 | C=O |
| 1171 | C-O |
| 1035 | C-O |

The spectrum shows the characteristic infrared signals of maltodextrins and, in addition, the absorption peak of carbonyl units, located at 1723 cm⁻¹, thus confirming the presence of the cross-linkers in the polymeric structure.

The polymer obtained was also subjected to CHNS analysis in a Thermoscientific FlashEA 1112 Series instrument. The results are reported in the table below:

| | % N | % C | % H | % S |
|---|---|---|---|---|
| Cross-linked polymer of example 6 | 0.00 | 38.91 | 4.27 | 0.00 |

### Example 7 Preparation of the cross-linked polymer of the invention by reacting a maltodextrin deriving from starch comprising amylose in the range from 25 to 50% expressed as dry weight relative to the dry weight of the starch and Citric acid as cross-linking agent

4.00 g of Linecaps Kleptose ® sold by Roquette Italia SpA and described in US2010/0196542, 0.40 of β-cyclodextrin, 1.00 g of sodium hypophosphite monohydrate (NaH₂PO₂•H₂O) and 14.22 g of citric acid were added to 20 ml of deionized water. The molar ratio between the maltodextrins and the cross-linking agent, i.e. Linecaps Kleptose ®and β-cyclodextrin with respect to citric acid was 1:2.73 expressed as molar ratio of one mole of glucose of the maltodextrin units with respect to 2.73 moles of citric acid. Whereas, the amount of β-cyclodextrin, introduced in the reaction, was equal to 10% w/w the amount of Linecaps Kleptose®. The solution was then heated at 100°C and maintained for 5 minutes until it was clear. The solution was then poured in a Petri dish, then maintained in stove at a pressure of about 80 mbar till the compound resulted to be dried (requested time: about 10 days). At the end of the synthesis, the polymer so obtained was ground through a pestle mortar and washed with deionized water in excess either through a Buchner funnel or through repeated centrifugation cycles. The treatment was continued until the washing water resulted to be colourless, then the treatment was continued with repeated rinses in acetone until the washing solvent was colourless. The polymer was then left to dry at the open air for a few days.

In order to characterize the cross-linked polymer of the invention, it was analyzed with ATR-FTIR analysis, employing a PerkinElmer Spectrum 100 FT-IR spectrometer. The result of the analysis is the spectrum reported in Figure 10. The principal absorption peaks are summarized in the subsequent table

| **Wave number (cm⁻¹)** | **Absorbing group** |
|---|---|
| 3600-3100 | O-H |
| 2990-2800 | C-H |
| 1716 | C=O |
| 1176 | C-O |
| 1012 | C-O |

Additionally to the main absorption bands of the glucose units, carried by both Linecaps and β-CD units, the characteristic absorption peak of carbonyl moieties appeared at 1716 cm⁻¹, thus confirming the presence of the cross-linking bridges in the polymer structure.

The polymer obtained was also subjected to CHNS analysis in a Thermoscientific FlashEA 1112 Series instrument. The results are reported in the table below:

| | % N | % C | % H | % S |
|---|---|---|---|---|
| Cross-linked polymer of example 7 | 0.00 | 39.66 | 4.36 | 0.00 |

### Example 8 Preparation of the cross-linked polymer of the invention by reacting a maltodextrin deriving from starch comprising amylose in the range from 25 to 50% expressed as dry weight relative to the dry weight of the starch and Citric acid as cross-linking agent

The same procedure and ingredients stated in Example 7 was repeated and resulted to be successful even by using an higher amount of β-cyclodextrin and, in some cases, by substituting sodium hypophosphite monohydrate (NaH₂PO₂•H₂O) with KH₂PO₄ according to the details reported below:

| Compound | Deionized water | Maltodextrin Linecaps | β-cyclodextrin | NaH₂PO₂•H₂O | KH₂PO₄ | Citric acid |
|---|---|---|---|---|---|---|
| Linecaps +20%βCD-CITR(1:3) | 20 ml | 4.00 g | 0.80 g | 1.00 g | - | 14.22 g |
| Linecaps +10%βCD-CITR(1:3) with KH₂PO₄ | 20 ml | 4.00 g | 0.40 g | - | 1.00 g | 14.22 g |
| Linecaps +20%βCD-CITR(1:3) With KH₂PO₄ | 20 ml | 4.00 g | 0.80 g | - | 1.00 g | 14.22 g |

### Example 9:

### Absorption of methyl orange.

The absorption of methyl orange, which is a pH indicator frequently used in titrations, was evaluated. Specifically the polymer of example 2 and example 3 were tested.

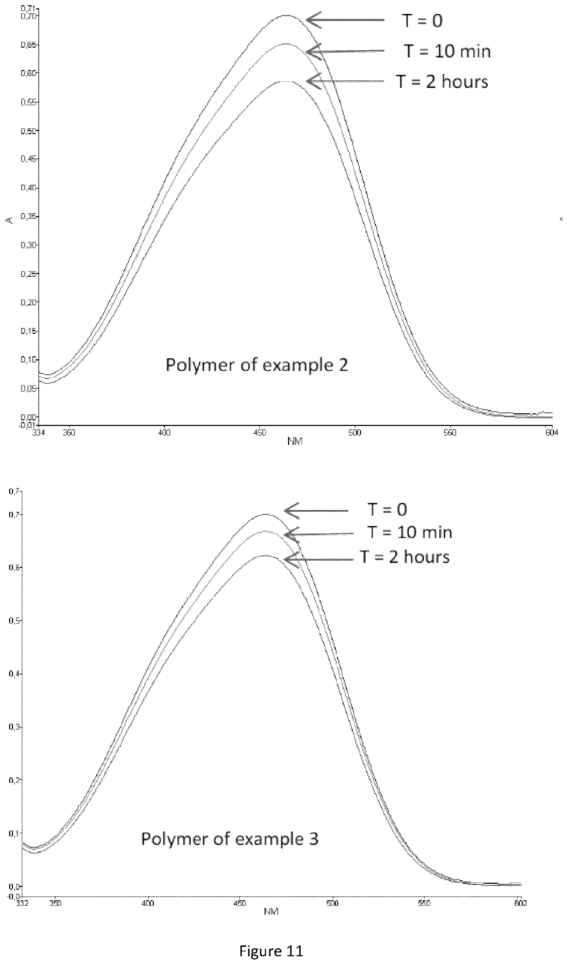

The absorption was evaluated by adding 50 mg of the polymer of example 2 and 50 mg of the polymer of Example 3, separately, to a 5ml of a solution of methyl orange (1.5x10⁻⁵ M) though the UV-Vis analysis of the concentration of methyl orange (peak at 464nm) in time. The results are graphically shown in Figure 11. As it is evident from Figure 11, for both the polymers of the invention the absorption of the methyl orange after two hours was evident.

Normalizing to 1 g of polymer for simplicity, it was observed that 1 g of the polymer of example 2, added to a solution of 0.500 mg of methyl orange in 100 ml of water, was able to absorb 0.036 mg (7.2%) of methyl orange after 10 minutes and 0.082 mg (16.4%) after 2 hours. Whereas, 1 g of the polymer of example 3, added to the same aqueous solution of methyl orange was able to absorb 0.024 mg (4.8%) of methyl orange after 10 minutes and 0.056 mg (11.2%) after 2 hours.

### Example 10

### Solubility of ketoprofen/dexamethasone owing to the encapsulation in the polymers of the invention.

The polymers of the examples 1 and 2 were tested in order to evaluate the the solubility of ketoprofene and dexamethasone (expressed as mg/ml) owing to the encapsulation in the polymers of the invention, when compared to the solubility in water, after encapsulation in Linecaps Kleptose ® sold by Roquette Italia SpA and described in US2010/0196542 and in hydroxypropyl-β-cyclodextrin. The results are reported in the following Table 2.

| | Water | Product Linecaps Kleptose | HP-β-CD | Polymer 2 | Polymer 1 |
|---|---|---|---|---|---|
| Ketoprofen | 0.22 | 0.39 | 10.36 | 1,40 | 4.60 |
| Dexamethasone | 0.09 | 0.60 | 7.21 | 1,53 | 5.0 |

It is evident the great enhancement in the solubility of the reported drugs by using cross-linked polymer of the invention. In particular polymer 1 surprisingly almost approaches the results got by HP-β-CD in the solubility of Dexamethasone.

### Example 11:

### Absorption of anisole.

The ability of the polymer of example 2 to absorb anisole, which is a precursor to pharmaceutical and perfumes, was studied.

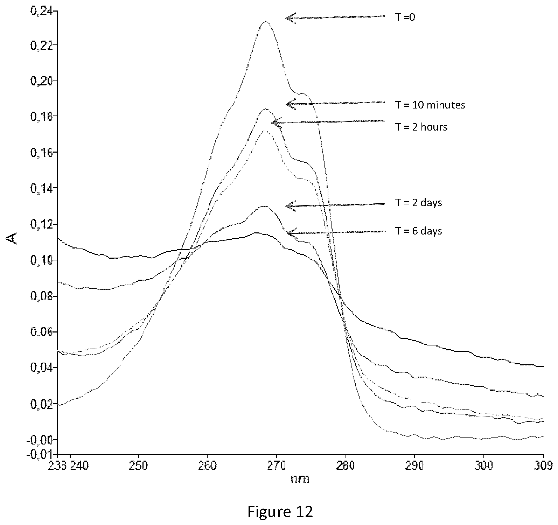

The absorption was evaluated by adding 50 mg of the polymer of example 2 to a 5ml of a solution of anisole (2.45x10⁻⁴ M) through the UV-Vis analysis of the concentration of anisole (peak at 268 nm) in time. The results are graphically shown in Figure 12.

Normalizing to 1 g of polymer for simplicity, it was observed that 1 g of the polymer of example 2, added to a solution of 2.644 mg of anisole in 100 ml of water, was able to absorb 0.550 mg (20.8%) of anisole after 10 minutes, 0.694 mg (26.2%) after 2 hours, 1.164 mg (44.0%) after 48 hours and 1.334 mg (50.5%) after 6 days.

## Claims

1. A cross-linked polymer obtainable by reacting a maltodextrin deriving from starch comprising amylose in the range from 25 to 50% expressed as dry weight relative to the dry weight of the starch and at least one cross-linking compound having a electropositive carbon atom selected from the group consisting of a dicarboxylic acid, dianhydride, carbonyldiimidazole, diphenylcarbonate, triphosgene, acylic dichloride, diisocyanate and diepoxide,
wherein the maltodextrin derives from leguminous starch and the leguminous plant is chosen from the group consisting of pea, bean, broad bean, horse bean and their mixtures.

2. The polymer according to claim 1, wherein the leguminous starch has an amylose content from 30% to 40%, preferably 35% to 40%, more preferably from 35% to 38%, these percentages being expressed as dry weight relative to the dry weight of starch.

3. The polymer according to claim 1 or 2, wherein the maltodextrin has a dextrose equivalent (DE) of 17.

4. The polymer according to anyone of claims 1-3, wherein the at least one crosslinking compound having a electropositive carbon atom is selected from a dicarboxylic acid, a dianhydride, carbonyldiimidazole and a diisocyanate.

5. The polymer according to anyone of claims 1-4, wherein the at least one crosslinking compound having a electropositive carbon atom is selected from pyromellitic dianhydride, 1,1'-carbonyldiimidazole, hexamethylene diisocyanate, citric acid and tartaric acid.

6. The polymer according to claim 4 or 5, wherein the at least one cross-linking compound having a electropositive carbon atom is citric acid.

7. The polymer according to anyone of claims 4-6, wherein the at least one cross-linking compound having a electropositive carbon atom is tartaric acid.

8. The polymer according to claim 4 or 5, wherein the at least one cross-linking compound having a electropositive carbon atom is pyromellitic dianhydride.

9. The polymer according to claim 4 or 5, wherein the at least one cross-linking compound having a electropositive carbon atom is hexamethylene diisocyanate.

10. The polymer according to claim 4 or 5, wherein the at least one cross-linking compound having a electropositive carbon atom is 1,1'-carbonyldiimidazole.

11. A process for preparing a cross-linked polymer according to anyone of the claims 1-10, comprising the following steps:
a) Preparing a solution of a maltodextrin deriving from starch comprising amylose in the range from 25 to 50% expressed as dry weight relative to the dry weight of the starch;
b) Adding and reacting at least one cross-linking compound having an electropositive carbon atom selected from the group consisting of a dicarboxylic acid, dianhydride, carbonyldiimidazole, diphenylcarbonate, triphosgene, acylic dichloride, diisocyanate and diepoxide;
c) Obtaining the polymer,
wherein the maltodextrin derives from leguminous starch and the leguminous plant is chosen from the group consisting of pea, bean, broad bean, horse bean and their mixtures.

12. The process according to claim 11, wherein the molar ratio between the maltodextrin of step a) and the at least one compound having an electropositive carbon is from 1:0.5 to 1:250.

13. The process according to claim 12, wherein the molar ratio between the maltodextrin of step a) and the at least one cross-linking compound having an electropositive carbon is 1: 0.57.

14. The process according to claim 12, wherein the molar ratio between the maltodextrin of step a) and citric acid is 1: 3.

15. The process according to claim 12, wherein the molar ratio between the maltodextrin of step a) and tartaric acid and citric acid is 1: 1:2.

16. The process according to claim 11, wherein a cyclodestrin, preferably β-cyclodextrin, is added in step b) together with the at least one cross-linking agent.

17. The process according to anyone of claims 11-16, the solution of step a) is carried out with dimethyl sulfoxide or with N,N-dimethylformamide or N-methlpyrrolidinone.

18. Use of the polymer according to anyone of claims 1-10 for the encapsulation/inclusion/entrapment of an organic compound.

## Patentansprüche

1. Vernetztes Polymer, das durch Reaktion von aus einem von Stärke stammenden Maltodextrin, das Amylose im Bereich von 25 bis 50% aufweist, angegeben als Trockengewicht bezüglich dem Trockengewicht der Stärke, mit zumindest einer Vernetzungsverbindung mit einem elektropositiven Kohlenstoffatom ausgewählt aus der Gruppe bestehend aus einer Dicarbonsäure, Dianhydrid, Carbonyldiimidazol, Diphenylcarbonat, Triphosgen, Azyldichlorid, Diisocyanat und Diepoxid erhalten wird,
wobei das Maltodextrin von Hülsenfruchtstärke stammt und die Hülsenfrucht ausgewählt wird aus der Gruppe bestehend aus Erdnuss, Bohne, Ackerbohne, Pferdebohne und Mischungen daraus.

2. Polymer gemäß Anspruch 1, wobei die Hülsenfruchtstärke einen Amylosegehalt zwischen 30% und 40%, vorzugsweise zwischen 35% und 40% und weiter bevorzugt zwischen 35% und 38%, wobei die Prozentangaben als Trockengewicht bezüglich dem Trockengewicht der Stärke angegeben sind.

3. Polymer gemäß Anspruch 1 oder 2, wobei das Maltodextrin ein Dextroseäquivalent (DE) von 17 aufweist.

4. Polymer gemäß einem der Ansprüche 1 bis 3, wobei die zumindest eine Vernetzungsverbindung mit einem elektropositiven Kohlenstoffatom ausgewählt wird aus einer Dicarbonsäure, einem Dianhydrid, Carbonyldiimidazol und einem Diisocyanat.

5. Polymer gemäß einem der Ansprüche 1 bis 4, wobei die zumindest eine Vernetzungsverbindung mit einem elektropositiven Kohlenstoffatom ausgewählt wird aus Pyromellitsäuredianhydrid, 1,1'-Carbonyldiimidazol, Hexamethylendiisocyanat Zitronensäure und Weinsäure.

6. Polymer gemäß Anspruch 4 oder 5, wobei die zumindest eine Vernetzungsverbindung mit einem elektropositiven Kohlenstoffatom Zitronensäure ist.

7. Polymer gemäß Anspruch 5, wobei die zumindest eine Vernetzungsverbindung mit einem elektropositiven Kohlenstoffatom Weinsäure ist.

8. Polymer gemäß Anspruch 4 oder 5, wobei die zumindest eine Vernetzungsmittel mit einem elektropositiven Kohlenstoffatom Pyromellitsäuredianhydrid ist.

9. Polymer gemäß Anspruch 4 oder 5, wobei die zumindest eine Vernetzungsverbindung mit einem elektropositiven Kohlenstoffatom Hexamethylendiisocyanat ist.

10. Polymer gemäß Anspruch 4 oder 5, wobei die zumindest eine Vernetzungsverbindung mit einem elektropositiven Kohlenstoffatom 1,1'-Carbonyldiimidazol ist.

11. Verfahren zur Herstellung eines vernetzten Polymers gemäß einem der Ansprüche 1 bis 10, folgende Schritte aufweisend:
a) Zubereiten einer Lösung eines von Stärke stammenden Maltodextrins, das Amylose im Bereich von 25 bis 50%, angegeben als Trockengewicht bezüglich dem Trockengewicht der Stärke, aufweist;
b) Zugabe und Reaktion mit zumindest einer Vernetzungsverbindung mit einem elektropositiven Kohlenstoffatom ausgewählt aus der Gruppe bestehend aus einer Dicarbonsäure, Dianhydrid, Carbonyldiimidazol, Diphenylcarbonat, Triphosgen, Azyldichlorid, Diisocyanat und Diepoxid;
c) Erhalten des Polymers,
wobei das Maltodextrin von Hülsenfruchtstärke stammt und die Hülsenfrucht ausgewählt wird aus der Gruppe bestehend aus Erdnuss, Bohne, Ackerbohne, Pferdebohne und Mischungen daraus.

12. Verfahren gemäß Anspruch 11, wobei das Molverhältnis zwischen dem Maltodextrin aus Schritt a) und der zumindest einen Verbindung mit einem elektropositiven Kohlenstoff zwischen 1 : 0,5 und 1 : 250 liegt.

13. Verfahren gemäß Anspruch 12, wobei das Molverhältnis zwischen dem Maltodextrin aus Schritt a) und der zumindest einen Vernetzungsverbindung mit einem elektropositiven Kohlenstoff 1 : 0,57 beträgt.

14. Verfahren gemäß Anspruch 12, wobei das Molverhältnis zwischen dem Maltodextrin aus Schritt a) und Zitronensäure 1 : 3 beträgt.

15. Verfahren gemäß Anspruch 12, wobei das Molverhältnis zwischen dem Maltodextrin aus Schritt a) und Weinsäure und Zitronensäure 1 : 1 : 2 beträgt.

16. Verfahren gemäß Anspruch 11, wobei ein Cyclodextrin, vorzugsweise β-Cyclodextrin, in Schritt b) zusammen mit dem zumindest einen Vernetzungsmittel zugegeben wird.

17. Verfahren gemäß einem der Ansprüche 11 bis 16, wobei die Lösung aus Schritt a) erhalten wird durch Verwendung von Dimethylsulfoxid oder N,N-Dimethylformamid oder N-Methylpyrrolidinon.

18. Verwendung des Polymers gemäß einem der Ansprüche 1 bis 10 zum Einkapseln / Einbinden / Einschließen einer organischen Verbindung.

## Revendications

1. Polymère réticulé apte à être obtenu par la mise en réaction d'une maltodextrine dérivée de l'amidon comprenant de l'amylose dans la plage de 25 à 50 %, exprimé en poids sec par rapport au poids sec de l'amidon, et d'au moins un composé de réticulation ayant un atome de carbone électropositif sélectionné parmi le groupe constitué d'un acide dicarboxylique, d'un dianhydride, d'un carbonyldiimidazole, d'un carbonate de diphényle, du triphosgène, d'un dichlorure d'acyle, d'un diisocyanate et d'un diépoxyde,
dans lequel la maltodextrine dérive de l'amidon de légumineuse et la plante légumineuse est choisie parmi le groupe constitué des pois, des haricots, des fèves, des féveroles et leurs mélanges.

2. Polymère selon la revendication 1, dans lequel l'amidon de légumineuse possède une teneur en amylose de 30 % à 40 %, de préférence de 35 % à 40 %, plus préférablement de 35 % à 38 %, ces pourcentages étant exprimés en poids sec par rapport au poids sec d'amidon.

3. Polymère selon la revendication 1 ou 2, dans lequel la maltodextrine possède un équivalent dextrose (DE) de 17.

4. Polymère selon l'une quelconque des revendications 1 à 3, dans lequel l'au moins un composé de réticulation ayant un atome de carbone électropositif est sélectionné parmi un acide dicarboxylique, un dianhydride, un carbonyldiimidazole et un diisocyanate.

5. Polymère selon l'une quelconque des revendications 1 à 4, dans lequel l'au moins un composé de réticulation ayant un atome de carbone électropositif est sélectionné parmi le dianhydride pyromellitique, le 1,1'-carbonyldiimidazole, le diisocyanate d'hexaméthylène, l'acide citrique et l'acide tartrique.

6. Polymère selon la revendication 4 ou 5, dans lequel l'au moins un composé de réticulation ayant un atome de carbone électropositif est l'acide citrique.

7. Polymère selon la revendication 5, dans lequel l'au moins un composé de réticulation ayant un atome de carbone électropositif est l'acide tartrique.

8. Polymère selon la revendication 4 ou 5, dans lequel l'au moins un composé de réticulation ayant un atome de carbone électropositif est le dianhydride pyromellitique.

9. Polymère selon la revendication 4 ou 5, dans lequel l'au moins un composé de réticulation ayant un atome de carbone électropositif est le diisocyanate d'hexaméthylène.

10. Polymère selon la revendication 4 ou 5, dans lequel l'au moins un composé de réticulation ayant un atome de carbone électropositif est le 1,1'-carbonyldiimidazole.

11. Procédé de préparation d'un polymère réticulé selon l'une quelconque des revendications 1 à 10, comprenant les étapes suivantes :
a) préparer une solution d'une maltodextrine dérivant de l'amidon comprenant de l'amylose dans la plage de 25 à 50 %, exprimé en poids sec par rapport au poids sec de l'amidon ;
b) ajouter et mettre en réaction au moins un composé de réticulation ayant un atome de carbone électropositif sélectionné parmi le groupe constitué d'un acide dicarboxylique, d'un dianhydride, d'un carbonyldiimidazole, d'un carbonate de diphényle, du triphosgène, d'un dichlorure d'acyle, d'un diisocyanate et d'un diépoxyde ;
c) obtenir le polymère,
dans lequel la maltodextrine dérive de l'amidon de légumineuse et la plante légumineuse est choisie parmi le groupe constitué des pois, des haricots, des fèves, des féveroles et leurs mélanges.

12. Procédé selon la revendication 11, dans lequel le rapport molaire entre la maltodextrine de l'étape a) et l'au moins un composé ayant un carbone électropositif est de 1:0,5 à 1:250.

13. Procédé selon la revendication 12, dans lequel le rapport molaire entre la maltodextrine de l'étape a) et l'au moins un composé de réticulation ayant un carbone électropositif est de 1: 0,57.

14. Procédé selon la revendication 12, dans lequel le rapport molaire entre la maltodextrine de l'étape a) et l'acide citrique est de 1: 3.

15. Procédé selon la revendication 12, dans lequel le rapport molaire entre la maltodextrine de l'étape a) et l'acide tartrique et l'acide citrique est de 1: 1:2.

16. Procédé selon la revendication 11, dans lequel une cyclodextrine, de préférence la β-cyclodextrine, est ajoutée à l'étape b) conjointement avec l'au moins un agent de réticulation.

17. Procédé selon l'une quelconque des revendications 11 à 16, la solution de l'étape a) est réalisée avec le diméthylsulfoxyde ou avec la N,N-diméthylformamide ou la N-méthylpyrrolidinone.

18. Utilisation du polymère selon l'une quelconque des revendications 1 à 10 pour l'encapsulation/l'inclusion/le piégeage d'un composé organique.
